# EUROPÄISCHE PATENTSCHRIFT

(11) **EP 1 728 777 B1**
(45) Veröffentlichungstag und Bekanntmachung des Hinweises auf die Patenterteilung: **16.09.2009**
(21) Anmeldenummer: 06010611.9
(22) Anmeldetag: 23.05.2006
(51) Int. Cl.: C07C 37/20, C07C 37/84, C07C 39/16

(54) **Verfahren zur Herstellung von 2,2-bis(4-hydroxyphenyl)Propan (Bisphenol A)**
Process for the preparation of 2,2-bis(4-hydroxyphenyl)propane (Bisphenol A)
Procédure pour la préparation du 2,2-bis(4-hydroxyphenyl)propane (Bisphenol A)

(30) Priorität: 04.06.2005 DE 102005025788
(43) Veröffentlichungstag der Anmeldung: 06.12.2006
(73) Patentinhaber: Bayer MaterialScience AG, 51368 Leverkusen (DE)
(72) Erfinder: Blaschke, Ulrich, Dr., 47829 Krefeld (DE); Westernacher, Stefan, Dr., 47906 Kempen (DE); Braun, Arne, 51381 Leverkusen (DE); Audenaert, Raymond, Dr., 9220 Hamme (BE); Zank, Jesko, Dr., 51061 Köln (DE)

(56) Entgegenhaltungen:
- US-A- 5 198 591
- US-A1- 2003 181 768
- US-B1- 6 710 211

## Beschreibung

Die Erfindung betrifft ein Verfahren zur Herstellung von hochreinem Bisphenol A.

2,2-Bis(4-hydroxyphenyl)propan (Bisphenol A, BPA) als Kondensationsprodukt von Phenol und Aceton ist Ausgangsstoff oder Zwischenprodukt zur Herstellung einer Vielzahl kommerzieller Produkte. So dient es als Ausgangsstoff zur Herstellung verschiedenartiger polymerer Werkstoffe wie beispielsweise Polyarylate, Polyetherimide, Polysulfone und modifizierter Phenol-Formaldehydharze. Bevorzugte Anwendungsgebiete liegen in der Herstellung von Epoxyharzen und Polycarbonaten.

Technisch relevante Herstellmethoden für BPA sind aus dem Stand der Technik, z.B. WO 00/35847 und US 2,775,620, bekannt.

Nach der sauer katalysierten Reaktion von Phenol mit Aceton wird BPA aus dem Produktgemisch in Form eines kristallinen Addukts von BPA und Phenol abgetrennt. Aus diesen BPA-Phenol-Adduktkristallen wird durch destillative, desorptive oder extraktive Methoden Phenol ganz oder teilweise entfernt. Die BPA-Phenol-Adduktkristalle können vor der Abtrennung von Phenol noch zusätzlichen Reinigungsschritten unterworfen werden, um eine Verringerung der Konzentration an Nebenkomponenten zu erzielen.

Die BPA-Phenol-Adduktkristalle können beispielsweise aus Phenol, organischen Lösungsmitteln oder Mischungen dieser Lösungsmittel umkristallisiert werden. Hierbei kann durch die Wahl geeigneter Lösungsmittel auch das in den Adduktkristallen vorhandene Phenol ganz oder teilweise abgetrennt werden.

So beschreibt PL 159620 ein aufwendiges Verfahren zur Aufreinigung von Bisphenol A, bei dem die in einer ersten Stufe isolierten BPA-Phenol-Adduktkristalle in Phenol gelöst werden, die erhaltene Lösung in einem diskontinuierlich betriebenen Kristaller in einer Art statischem Schichtkristallisationsverfahren abgekühlt wird, wobei BPA-Phenol-Adduktkristalle auf den Rohren aufwachsen. Nach Ablassen der Mutterlauge werden die derart umkristallisierten BPA-Phenol-Adduktkristalle mit wasserhaltigem Phenol gewaschen, um noch anhaftende Mutterlauge zu entfernen, und/oder durch Erwärmen auf eine Temperatur von 75 bis 85°C und somit durch Ausschwitzen weiter aufgereinigt. Da das Verfahren aber diskontinuierlich betrieben werden muss, ist der apparative Aufwand entsprechend hoch. Außerdem sind in diesem Verfahren die auf die Wärmeaustauscherfläche bezogenen Produktionsleistungen um Größenordnung niedriger als bei einer kontinuierlichen Suspensionskristallisation von BPA-Phenol-Adduktkristallen. Darüber hinaus sind für eine akzeptable Raum-Zeit-Ausbeute hohe Kühlraten erforderlich, so dass eine derartige Schichtkristallisation im Vergleich zur kontinuierlichen Suspensionskristallisation bei wesentlich höheren Kristallwachstumsgeschwindigkeiten erfolgen muss. Der hierdurch verursachte verstärkte Einbau von Verunreinigungen in die Kristalle muss durch zeitintensive Schwitz- und/oder Waschvorgänge kompensiert werden. Bei hohen Reinheitsanforderungen muss unter Umständen ein mehrstufiges Verfahren eingesetzt werden.

EP 718 268 A beschreibt ein Verfahren zur Herstellung eines kristallinen Adduktes von Bisphenol A und Phenol, bei dem in Phenol gelöstes BPA zunächst als Addukt auskristallisiert und abfiltriert, anschließend erneut in Phenol gelöst, erneut auskristallisiert und abfiltriert wird, wobei dieser Schritt des Umkristallisierens mehrmals wiederholt wird. Im letzten Schritt der mehrstufigen Umkristallisation wird das Addukt mit speziell gereinigtem Phenol gewaschen. Zumindest teilweise werden die Waschflüssigkeiten und Filtrate für die Wäsche und Auflösung der Addukte rückgeführt.

WO 02/40435 beschreibt einen Prozess, bei dem hochreines Bisphenol A durch mehrfaches Umkristallisieren aus Phenol und einer Wäsche der Addukte in den einzelnen Stufen mit hochreinem Phenol in einer Kreuz-Strom-Wäsche erhalten wird. Das als Waschphenol eingesetzte Phenol enthält dabei quasi kein Bisphenol A oder seine Isomere. Eine mehrfache Umkristallisation wird bevorzugt. Durch die Wäsche mit hochreinem Phenol auf jeder der einzelnen Stufen wird sehr viel des hochreinen Phenols benötigt, darüber hinaus wird auf jeder einzelnen Stufe viel Bisphenol A in dem Phenol gelöst, es kommt zu Ausbeuteverlusten.

WO 03/82785 beschreibt ein Verfahren zur Herstellung von Bisphenol A, bei dem im ersten Schritt eine Suspension von BPA-Phenol-Adduktkristallen unter Vakuum filtriert und gewaschen wird, der erhaltene Filterkuchen in einer Phenol enthaltenden Flüssigkeit aufgelöst und erneut kristallisiert wird. Die Abtrennung der so erhaltenen Kristalle erfolgt mittels Zentrifugation. Die für größere Anlagenkapazitäten wirtschaftlicheren kontinuierlicharbeitenden (Filter-)zentrifugen benötigen allerdings eine minimale Kristallgröße, die bestimmte Fahrweisen in der Kristallisation erfordert.

EP 1 367 043 A beschreibt ein Verfahren zur Aufreinigung von Bisphenol A, bei dem die BPA-Phenol-Adduktkristalle in Phenol gelöst werden und die resultierende Lösung mindestens einmal filtriert wird, bevor die BPA-Phenol-Adduktkristalle erneut auskristallisiert werden.

Aufgabe der vorliegenden Erfindung ist es, ein einfaches Verfahren zur Herstellung von hochreinem Bisphenol, insbesondere Bisphenol A, mit einer Reinheit mindestens 99,8% zur Verfügung zu stellen, wobei eine niedrige Farbzahl und eine hohe Temperaturstabilität in der Schmelze erhalten werden.

Unter einer niedrigen Farbzahl versteht man dabei eine Farbzahl von maximal 20 Hazen. Zur Bestimmung der Farbzahl werden 10 g Bisphenol A unter Luft in einem Ölbad bei einer Temperatur von 175°C innerhalb von 20 Minuten aufgeschmolzen und dann sofort die Farbzahl gemäß ASTM D 1209 bestimmt. Zur Bestimmung der Temperaturstabilität wird dieses Material anschließend für 4,5 h bei 175°C Badtemperatur erhitzt und anschließend erneut die Farbzahl gemessen. Unter einer hohen Temperaturstabilität versteht man dabei einen Anstieg um maximal 40 Hazen.

Gegenstand der Erfindung ist ein Verfahren zur Herstellung von Bisphenol A umfassend die folgenden Schritte:
a) Umsetzung von Phenol und Aceton in Gegenwart eines sulfonsauren Ionenaustauschers und eines schwefelhaltigen Cokatalysators zu einem Bisphenol A enthaltenden Produktgemisch
b) kontinuierliche Suspensionskristallisation eines Bisphenol A-Phenol-Addukts aus dem in Schritt (a) erhaltenen Produktgemisch, welches 0,1 bis 6 Gew.-%, bevorzugt 0,5 bis 2 Gew.-%, Wasser enthält, in mindestens einem Kristallisator mit jeweils mindestens einem Wärmetauscher
c) Abtrennung der gemäß Schritt (b) gewonnenen Bisphenol A-Phenol-Adduktkristalle durch Fest-Flüssig-Trennung, insbesondere durch Filtration, und Waschen der Feststoffphase, insbesondere des Filterkuchens, mit phenolischer Lösung sowie anschließende destillative Abtrennung von Wasser aus den Flüssigphasen, insbesondere aus den Filtraten, der Fest-Flüssig-Trennung und/oder der Wäsche auf einen Gehalt von maximal 0,3 Gew.-% Wasser und Rückführung der so entwässerten Lösung zu mindestens 90 Gew.-% in Schritt (a), wobei die rückgeführte Lösung einen Gehalt von 5 bis 15 Gew.-%, bevorzugt 6,5 bis 10 Gew.-% p,p'-BPA und 3 bis 12 Gew.-% Isomere, aufweist
d) Herstellen einer homogenen Lösung, enthaltend 15 bis 35 Gew.-%, bevorzugt 20 bis 30 Gew.-%, Bisphenol A, 0,05 bis 2 Gew.-%, bevorzugt 0,1 bis 1,1 Gew.-%, Isomere und 0,1 bis 10 Gew.-% Wasser, in Phenol aus der in Schritt (c) erhaltenen Feststoffphase, insbesondere aus dem Filterkuchen, unter Zugabe einer phenolischen Lösung
e) kontinuierliche Suspensionskristallisation eines Bisphenol A-Phenol-Addukts aus der in Schritt (d) erhaltenen Lösung in mindestens einem Kristallisator mit jeweils mindestens einem Wärmetauscher
f) Abtrennung der gemäß Schritt (e) gewonnenen Bisphenol A-Phenol-Adduktkristalle durch Fest-Flüssig-Trennung, insbesondere durch Filtration, und Waschen der Feststoffphase, insbesondere des Filterkuchens, mit phenolischer Lösung
g) Entfernung von Phenol aus dem gemäß Schritt (f) erhaltenen BPA-Phenol-Addukt durch Erhitzen auf Temperaturen von mindestens 120°C.

Bei dem erfindungsgemäßen Verfahren wird ein Bisphenol A mit einer Reinheit von mindestens 99,8 Gew.-% an p,p-BPA bezogen auf alle im Produkt enthaltenen Komponenten außer Phenol erhalten.

In Schritt (a) des erfindungsgemäßen Verfahrens erfolgt die Umsetzung von Phenol und Aceton in Gegenwart eines sulfonsauren Ionenaustauschers und eines schwefelhaltigen Cokatalysators zu einem Bisphenol A enthaltenden Produktgemisch. Schritt (a) beruht auf der säurekatalysierten Umsetzung von Phenol mit Aceton, wobei bevorzugt ein Phenol-Aceton-Verhältnis von mindestens 5 : 1 in der Reaktion eingestellt wird. Die Reaktion erfolgt dabei üblicherweise im Dauerbetrieb und im allgemeinen bei Temperaturen von 45 bis 110°C, bevorzugt bei 50 bis 80°C. Als saure Katalysatoren kommen gelförmige (mikroporöse) oder makroporöse sulfonierte vernetzte Polystyrolharze (saure Ionentauscher) zum Einsatz, die sowohl monodispers als auch heterodispers sein können. Als Vernetzer wird normalerweise Divinylbenzol eingesetzt, aber auch andere Vernetzer wie Divinylbiphenyl können Verwendung finden. Neben dem Katalysator kommt ein Cokatalysator zum Einsatz. Hierbei handelt es sich üblicherweise um ein Thiol, das mindestens eine SH-Funktion trägt und sowohl die Selektivität als auch die Reaktivität der Reaktion positiv beeinflusst. Der Cokatalysator kann sowohl homogen in der Reaktionslösung gelöst als auch auf dem Katalysator selber fixiert sein. Homogene Cokatalysatoren sind beispielsweise Mercaptopropionsäure, Schwefelwasserstoff, Alkylsulfide oder Alkylsilylthiole wie beispielsweise Ethylsulfid oder Silylmethanthiol und ähnliche Verbindungen. Fixierte Cokatalysatoren sind Aminoalkylthiole und Pyridylalkylthiole, die ionisch an den Katalysator gebunden sind, wobei die SH-Funktion geschützt sein kann und erst während oder nach Fixierung auf den Katalysator freigesetzt wird, wie beispielsweise bei Dimethylthiazolidin und bei Alkylcarbamoylalkylthioestern. Ebenso kann der Cokatalysator kovalent als Alkyl- oder Arylthiol an den Katalysator gebunden bzw. Bestandteil des Katalysators sein. Es können auch zwei oder mehrere der beschriebenen Cokatalysatoren gemeinsam zum Einsatz kommen.

Bei der Umsetzung von Phenol mit Aceton in Gegenwart saurer Katalysatoren entsteht eine Produktmischung, die neben nicht umgesetztem Phenol und gegebenenfalls Aceton im Wesentlichen BPA und Wasser enthält. Daneben treten in geringen Mengen Isomere als typische Nebenprodukte der Kondensationsreaktion auf, so beispielsweise 2-(4-Hydroxyphenyl)-2-(2-hydroxyphenyl)-propan (o,p-BPA), substituierte Indane, Hydroxyphenylindanole, Hydroxyphenylchromane, Spirobisindane, substituierte Indenole, substituierte Xanthene und höher kondensierte Verbindungen mit drei oder mehr Phenylringen im Molekülgerüst. Außerdem können sich durch Eigenkondensation des Acetons und Reaktion mit Verunreinigungen in den Rohstoffen weitere Nebenkomponenten wie Anisol, Mesityloxid, Mesitylen und Diacetonalkohol bilden.

Die Umsetzung gemäß Schritt (a) kann so durchgeführt werden, dass das Aceton vollständig umgesetzt wird. Aus wirtschaftlichen und technischen Gründen wird sie aber meist so gefahren, dass kein hundertprozentiger Umsatz des Acetons erreicht wird, sondern noch bis zu 1,0 Gew.-% Aceton im Reaktorablauf enthalten sind.

Die Reaktion kann auch in mehreren, in Reihe geschalteten Reaktoren erfolgen, wobei die Gesamtmenge des Acetons derart verteilt wird, dass vor Eintritt des Reaktionsgemisches in den jeweiligen Reaktor erneut Aceton zudosiert wird.

Die genannten Nebenprodukte aber auch die nicht umgesetzten Einsatzstoffe wie Phenol und Aceton beeinträchtigen die Eignung von BPA zur Herstellung von Polymeren und müssen durch geeignete Verfahren abgetrennt werden, da insbesondere zur Herstellung von Polycarbonat hohe Reinheitsanforderungen an das BPA gestellt werden.

Aus dem in Schritt (a) erhaltenen Produktgemisch, welches 0,1 bis 6 Gew.-%, bevorzugt 0,5 bis 2 Gew.-%, Wasser enthält, werden gemäß Schritt (b) durch kontinuierliche Suspensionskristallisation Bisphenol A-Phenol-Adduktkristalle auskristallisiert. Das in Schritt (a) erhaltene Produktgemisch wird vor Schritt (b) oder (c) keiner Destillation zur Entfernung von leichtflüchtigen Bestandteilen (einschließlich Wasser) unterworfen.

Die Kristallisation kann in einem oder mehreren, hintereinander geschalteten Kristallisatoren (nachfolgend auch als Kristaller bezeichnet) stattfinden. Die mittlere Gesamtverweilzeit der Kristalle in dieser Kristallisationsstufe beträgt bevorzugt 30 Minuten bis zehn Stunden. Bei einer entsprechend langsamen Kristallisation können der Einschluss von Mutterlauge sowie der Einbau von Verunreinigungen in die BPA-Phenol-Adduktkristalle weitestgehend verhindert werden. Die Kühlung erfolgt dabei indirekt durch Wärmeaustauscher. Bei der Kristallisation wird das Produktgemisch in einem Kristallisationsschritt auf 40 bis 50°C abgekühlt. Bevorzugt erfolgt die Kristallisation aber in zwei Schritten, wobei im ersten Schritt das Produktgemisch auf eine Temperatur von 50 bis 65°C, im zweiten Schritt dann auf eine Temperatur von 40 bis 50°C abgekühlt wird. In beiden Ausführungsformen erfolgt die Kühlung mittels einem oder mehreren Wärmetauschern pro Kristaller.

In einer bevorzugten Ausführungsform wird die Temperatur des Produktgemisches vor Schritt (b) auf eine Temperatur eingestellt, die maximal 5°C über dem Kristallisationspunkt liegt.

Die anschließende Fest-FlüssigTrennung, insbesondere Filtration, und Wäsche gemäß Schritt (c) kann z.B. in bzw. auf einem der folgenden Apparatetypen durchgeführt werden: kontinuierliche Filterzentrifugen, wie Siebschneckenzentrifugen oder Schubzentrifugen, diskontinuierliche Filterzentrifugen, wie Schälzentrifugen, kontinuierliche Filter, wie Dreh- oder Trommelfilter, Bandfilter und Scheibenfilter. Bevorzugt kommen Drehfilter, besonders bevorzugt Vakuumdrehfilter, zum Einsatz.

Bei der Filtration mittels eines Vakuumdrehfilters erfolgt die Filtration bevorzugt wie in DE 199 61 521 A beschrieben.

Die Wäsche der Feststoffphase, insbesondere des Filterkuchens, erfolgt mit einer phenolischen Lösung, wodurch die im Filterkuchen verbleibende Mutterlauge weitgehend verdrängt und die BPA-Phenol-Adduktkristalle von oberflächlich anhaftenden Verunreinigungen befreit werden. Der Filterkuchen kann mit einer einzigen oder mit mehreren phenolischen Lösungen unterschiedlicher Zusammensetzungen, die nacheinander oder gemischt, z.B. über Sprühdüsen, aufgegeben werden, gewaschen werden. Gemittelt über die Gesamtmenge der eingesetzten phenolischen Lösung enthält diese vorzugsweise 84 bis 99,45 Gew.-% Phenol, 0,5 bis 15 Gew.-% BPA, 0,05 bis 1,1 Gew.-% Isomere. Diese Konzentrationen beziehen sich auf die Lösung ohne Berücksichtigung eines Anteils an Wasser. Die eingesetzte phenolische Lösung enthält 0,05 bis 12 Gew.-% Wasser, bevorzugt 0,2 bis 3 Gew.-% Wasser, bezogen auf die Gesamtmenge (der phenolischen Lösung) und stammt vorzugsweise zu 5 bis 100 Gew.-% aus Schritt (f). Die Temperatur der Waschflüssigkeit liegt bevorzugt bei 40 bis 85°C, besonders bevorzugt bei 45 bis 70°C.

Im Falle eines Drehfilters kann ein Teil der phenolischen Lösung aus Schritt (f) für die Tuchspülung des Drehfilters in Schritt (c) verwendet werden.

Die gemäß Schritt (c) in Form eines phenolfeuchten Filterkuchens erhaltenen Adduktkristalle weisen bevorzugt eine Reinheit von mindestens 99 Gew.-% BPA, bezogen auf die Summe aus BPA und den Nebenkomponenten auf. Die Restfeuchte des Filterkuchens als Massenanteil der an den BPA-Phenol-Adduktkristallen anhaftenden Flüssigkeit bezogen auf die Gesamtmasse des feuchten Filterkuchens liegt bei unter 40 Gew.-%, bevorzugt bei 15 bis 30 Gew.-%. Bevorzugt wird die Menge an phenolischer Lösung zur Wäsche der abfiltrierten Bisphenol A-Phenol-Adduktkristalle so gewählt, dass die Waschflüssigkeitsmenge 20 bis 120 Gew.-% der abfiltrierten Menge an BPA-Phenol-Adduktkristallen entspricht. Bevorzugt werden dabei 50 bis 90 Gew.-% verwendet.

Vorzugsweise wird aus der in Schritt (c) erhaltenen Flüssigphase der Fest-Flüssig-Trennung und/oder der Wäsche das Wasser durch Destillation ganz oder teilweise, insbesondere bis auf einen Gehalt von 0 bis 0,3 Gew.-%, entfernt, und danach zu mindestens 90 Gew.-% ggf. nach Zusatz von Aceton, Phenol und/oder eines homogenen Cokatalysators in Schritt (a) zurückgeführt. Die rückgeführte Lösung enthält bevorzugt 5 bis 15 Gew.-%, besonders bevorzugt 6,5 bis 10 Gew.-%, p,p'-BPA und 3 bis 12 Gew.-% Isomere.

Aus den gemäß Schritt (c) erhaltenen BPA-Phenol-Adduktkristallen wird im nächsten Schritt (d) durch Zugabe einer phenolischen Lösung eine homogene Lösung, enthaltend u.a. 15 bis 35 Gew.-%, bevorzugt 20 bis 30 Gew.-%, BPA und 0,05 bis 2 Gew.-%, bevorzugt 0,1 bis 1,1 Gew.-%, Isomere, hergestellt. Dazu werden die BPA-Phenol-Adduktkristalle mit einer phenolischen Lösung vermischt, wobei die Temperatur der dabei entstehenden Lösung bevorzugt 70 bis 100°C beträgt und so eingestellt wird, dass die homogene Lösung untersättigt ist.

In einer bevorzugten Ausführungsform werden die phenolfeuchten BPA-Phenol-Adduktkristalle aus Schritt (c) zunächst aufgeschmolzen, wobei eine homogene Schmelze mit einer Temperatur von 90 bis 140°C, bevorzugt von 95 bis 130°C, erhalten wird. Anschließend wird eine phenolische Lösung hinzugemischt. Durch dieses Verfahren ist eine gute Durchmischung und eine zügige Herstellung der BPA-Phenol-Lösung erreicht. Ebenso wird vermieden, dass einzelne, verunreinigte Kristalle aus dem ersten Kristallisationsschritt sich nicht auflösen und zu einer Verunreinigung des Endproduktes führen.

Die Menge der in Schritt (d) eingesetzten phenolischen Lösung wird derart eingestellt, dass die resultierende homogene Lösung einen Gehalt an p,p'-BPA von 15 bis 35 Gew.-%, bevorzugt 20 bis 30 Gew.-%, aufweist. Die Konzentration an Isomeren liegt bei 0,05 bis 2 Gew.-%, bevorzugt bei 0,1 bis 0,8 Gew.-%, besonders bevorzugt bei 0,2 bis 0,5 Gew.-%. Diese Konzentrationen beziehen sich auf die Lösung ohne Berücksichtigung des Anteils an Wasser.

Die in Schritt (d) hergestellte homogene Lösung von BPA in Phenol enthält bevorzugt 0,1 bis 10 Gew.-% Wasser, besonders bevorzugt 0,2 bis 3 Gew.-% Wasser. Würde die homogene Lösung kein Wasser enthalten, würden im nachfolgenden Schritt der Kristallisation bei den niedrigen Konzentrationen an Isomeren in der Mutterlauge verstärkt nadelförmige Kristalle erhalten, die zu verschiedenen Problemen in der Anlage führen können, u.a. zu beschleunigtem Fouling an Kühlflächen bei der Kristallisation und zur Verschlechterung des. Auswaschverhaltens bei der Fest-Flüssigtrennung. Dies kann eine Beeinträchtigung der Produktqualität verursachen. Durch die Gegenwart von Wasser werden gedrungene, d.h. kürzere und dickere Kristalle erhalten. Außerdem bewirkt die Gegenwart von Wasser in diesen Konzentrationen während der Kristallisation einen verringerten Einbau von Verunreinigungen in die Kristalle. Dadurch lässt sich die Produktreinheit weiter steigern.

In einer weiteren erfindungsgemäßen Ausführungsform kann unabhängig von den Arbeitstemperaturen in den Kristallisatoren als weiterer Freiheitsgrad zur Zugabe von Wasser die Zugabe von Aceton in Konzentrationen von 0 bis 5 Gew.-%, bezogen auf das resultierende Gemisch, erfolgen. Mit dieser Maßnahme können u.a. die Kristallmorphologie, das Fest-Flüssig-Trennverhalten und die Foulingneigung, das Einbauverhalten der Verunreinigungen in die Adduktkristalle optimiert sowie die Konzentration der Mutterlauge eingestellt werden.

Anschließend wird gemäß Schritt (e) eine kontinuierliche Suspensionskristallisation eines BPA-Phenol-Addukts aus der in Schritt (d) erhaltenen homogenen Lösung durchgeführt. Aus der in Schritt (d) erhaltenen homogenen Lösung werden gemäß Schritt (e) durch kontinuierliche Suspensionskristallisation Bisphenol A-Phenol-Adduktkristalle gewonnen, welche durch Fest-Flüssig-Trennung, insbesondere Filtration, von der Flüssigphase abgetrennt und anschließend mit einer phenolischen Lösung gewaschen werden (Schritt (f)). Die Kühlung in Schritt (e) erfolgt indirekt durch Wärmeaustauscher.

Die Kristallisation kann in einem oder mehreren, hintereinander geschalteten Kristallern stattfinden. Die mittlere Gesamtverweilzeit der Kristalle in dieser Kristallisationsstufe sollte bevorzugt zwischen einer halben und zehn Stunden liegen. Bei einer entsprechend langsamen Kristallisation können der Einschluss von Mutterlauge sowie der Einbau von Verunreinigungen in die BPA-Phenol-Adduktkristalle weitestgehend verhindert werden. Bei der Kristallisation kann das Produktgemisch beispielsweise in einem Kristallisationsschritt auf 35 bis 55°C abgekühlt werden, wobei einer oder mehrere Kristaller parallel betrieben werden können. Alternativ dazu kann die Kristallisation in zwei Schritten erfolgen, wobei im ersten Schritt das Produktgemisch auf eine Temperatur von 45 bis 70°C, im zweiten Schritt dann auf eine Temperatur von 35 bis 55°C abgekühlt wird. In beiden Ausführungsformen erfolgt die Kühlung mittels einem oder mehreren Wärmetauschern pro Kristaller.

Eine alternative Ausführungsform der beiden Kristallisationsstufen (b) und (e) sieht folgendermaßen aus: In der ersten Kristallisationsstufe (Schritt (b)) erfolgt die Kristallisation seriell in zwei Schritten, in der zweite Stufe (Schritt (e)) in einem Schritt in einem oder mehreren parallel betriebenen Kristallern. Unter einem Schritt innerhalb einer Kristallisationsstufe wird eine Kristallisation in einem bestimmten Temperaturbereich verstanden. Eine seriell in zwei Schritten durchgeführte Kristallisationsstufe wird somit aufeinanderfolgend in zwei unterschiedlichen Temperaturbereichen durchgeführt. Durch das schrittweise Kristallisieren in der ersten Stufe wird eine größere Reinheit erzielt, während in der zweiten , Stufe aufgrund der dort herrschenden niedrigeren Isomerengehalte und der damit verbundenen stärkeren Foulingneigung eine größere Standzeit der Wärmetauscher durch Kristallisieren in einem Schritt erreicht wird.

In einer bevorzugten Ausführungsform wird die Temperatur des Produktgemisches vor Schritt (e) auf eine Temperatur eingestellt, die maximal 5°C über dem Kristallisationspunkt liegt.

Die anschließende Fest-Flüssigtrennung, insbesondere die Filtration, und Wäsche gemäß Schritt (f) wird z.B. in bzw. auf einem der folgenden Apparatetypen durchgeführt: kontinuierliche Filterzentrifugen, wie Siebschneckenzentrifugen oder Schubzentrifugen, diskontinuierliche Filterzentrifugen, wie Schälzentrifugen, kontinuierliche Filter, wie Dreh- oder Trommelfilter, Bandfilter und Scheibenfilter. Bevorzugt kommen Drehfilter, besonders bevorzugt Vakuumdrehfilter, zum Einsatz. Insbesondere bevorzugt ist die Verwendung eines Apparate-Typs, der auch in Schritt (c) zum Einsatz kommt.

Bei der Filtration mittels eines Vakuumdrehfilters erfolgt die Filtration bevorzugt wie in DE 199 61 521 A beschrieben.

Die Wäsche der Feststoffphase, insbesondere des Filterkuchens, erfolgt mit einer phenolischen Lösung, wodurch die im Filterkuchen verbleibende Mutterlauge weitgehend verdrängt und die Adduktkristalle von oberflächlich anhaftenden Verunreinigungen befreit werden. Die Feststoffphase, insbesondere der Filterkuchen, kann mit einer einzigen oder mit mehreren phenolischen Lösungen, die nacheinander oder gemischt, z. B. über Sprühdüsen, aufgegeben werden, gewaschen werden.

Das für die Wäsche der umkristallisierten BPA-Phenol-Adduktkristalle gemäß Schritt (f) eingesetzte phenolische Lösung kann sowohl Frischphenol, d.h. kommerziell erhältliches Phenol, als auch Rückphenole, die in der Anlage anfallen, sowie Mischungen beider sein. Unter einem Rückphenol wird ein Phenol verstanden, das im Laufe des Verfahrens anfällt und in das Verfahren rückgeführt wird. Es können auch Phenole verwendet werden, die bei der Herstellung von Polycarbonat nach dem Schmelzeverfahren anfallen. Bevorzugt enthält das hier eingesetzte Waschphenol in Summe maximal 1,0 Gew.-% an anderen phenolischen Komponenten wie z.B. Isopropenylphenol, BPA und seinen Isomeren bzw. Nebenkomponenten. Rückphenole, z.B. aus der Phenolentfernung aus den BPA-Phenol-Adduktkristallen, werden hierbei wenn überhaupt nur durch Destillation aufgereinigt. Die Temperatur des Waschphenols liegt bei 41 bis 75°C, bevorzugt bei 45 bis 60°C. Das Waschphenol kann bis zu 10 Gew.-% Wasser enthalten.

Die gemäß Schritt (f) in Form einer phenolfeuchten Feststoffphase, insbesondere eines phenolfeuchten Filterkuchens, erhaltenen Adduktkristalle weisen bevorzugt ein Reinheit von mindestens 99,8 Gew.-% BPA, bezogen auf die Summe aus BPA und den Nebenkomponenten auf. Die Restfeuchte des Filterkuchens als Massenanteil der an den BPA-Adduktkristallen anhaftenden Flüssigkeit bezogen auf die Gesamtmasse des feuchten Filterkuchens liegt bei unter 40 Gew.-%, Bevorzugt bei 15 bis 30 Gew.%. Bevorzugt wird die Menge an phenolischer Lösung zur Wäsche der abfiltrierten Bisphenol A-Phenol-Adduktkristalle so gewählt, dass die Waschflüssigkeitsmenge 20 bis 120 Gew.-% der abfiltrierten Menge an BPA-Phenol-Adduktkristallen entspricht. Bevorzugt werden dabei 50 bis 90 Gew.% verwendet.

Die Flüssigphase, insbesondere das Filtrat, der Fest-Flüssig-Trennung der zweiten Kristallisationsstufe (f) werden ggf. nach Zusatz von Wasser und/oder Aceton in Schritt (d) zur Vermischung mit aufgeschmolzenem Filterkuchen aus (c) sowie in Schritt (c) zur Wäsche des Filterkuchens und/oder für Spülzwecke (z.B. Tuchwäsche) eingesetzt. Vorzugsweise werden 10 bis 50 Gew.%, bevorzugt 20 bis 40 Gew.% der in Schritt (f) erhaltenen Flüssigphase zur Durchführung der Wäsche in Schritt (c) rückgeführt. Ferner werden bevorzugt 50 bis 90 Gew.-%, besonders bevorzugt 60 bis 80 Gew.-% der in Schritt (f) erhaltenen Filtrate dabei in Schritt (d) verwendet.

Die in Schritt (f) erhaltene Mutterlauge sowie die Wasch- und Entfeuchtungsfiltrate können in einer bevorzugten Ausführungsform ganz oder teilweise getrennt aufgefangen werden. Die weniger mit Isomeren verunreinigten Filtrate aus Wäsche und Entfeuchtung werden dabei bevorzugt in Schritt (d) geleitet, während ein Teil der Mutterlauge bevorzugt in Schritt (c) geführt wird. Durch die verstärkte Ausspeisung der Isomere sinkt deren Konzentrationsniveau in der zweiten Kristallisationsstufe, wodurch eine Verbesserung der Produktreinheit erreicht wird.

Unter Entfeuchtungsfiltrate sind die Filtrate zu verstehen, die bei Anlegen eines Vakuums aus dem Filterkuchen als Restwaschflüssigkeit entfernt werden.

Als weitere Möglichkeit, die Isomerenkonzentration in der zweiten Kristallisationsstufe (e) zu senken und damit die resultierende Produktreinheit zu erhöhen, besteht darin, 0 bis 35 Gew.-%, bevorzugt 0 bis 10 Gew.% der in Schritt (f) anfallenden Filtrate einzudampfen, das Bisphenol A und Isomeren-freie Kopfprodukt, das im Wesentlichen aus Phenol, Wasser und ggf. Aceton besteht, nach ggf. weiterer Aufreinigung in Schritt (d) und/oder Schritt (f) zu leiten und das an Bisphenol A und Isomeren angereicherte Sumpfprodukt vor Schritt (b) zurückzuführen. Durch diese Maßnahme wird die Phenolbilanz der Anlage besser kontrollierbar, was eine Verbesserung der Betreibbarkeit mit sich bringt.

Eine alternative Ausführungsform des Verfahrens beinhaltet die Herstellung von BPA zweier Reinheitsklassen, einem BPA mit einer Reinheit von 99,5 bis 99,75 Gew.-% und einem hochreinen BPA mit einer Reinheit von mindestens 99,8 Gew.%, in einer Anlage. Dabei werden sowohl die einstufige als auch die zweistufige Kristallisation sowie die anschließende Produktaufarbeitung (Fest-Flüssigtrennung, Wäsche, Lösen sowie Entfernen von Phenol) getrennt für die unterschiedlichen Reinheiten des BPA durchgeführt. Die Mutterlaugen der jeweils ersten Kristallisationsstufe und Fest-Flüssigtrennungsstufe können jedoch gemeinsam aufbereitet und in die Reaktion zurückgeführt werden, wobei das in der Reaktion gebildete Produktgemisch anschließend der getrennten Kristallisation und Produktaufarbeitung für die unterschiedlichen Reinheiten an BPA zugeführt wird.

In Schritt (g) wird Phenol aus der gemäß Schritt (f) erhaltenen Feststoffphase, insbesondere aus dem Filterkuchen, aus BPA-Phenol-Addukten durch thermische Trennung bei Temperaturen von mindestens 120°C entfernt. Die vollständige oder teilweise Entfernung des Phenols erfolgt bevorzugt durch destillative und/oder desorptive Methoden, wie beispielsweise in DE 198 48 026 A, DE 198 60 144 A und DE 199 61 566 A beschrieben.

Mit dem erfindungsgemäßen Verfahren lässt sich Bisphenol A mit einer Reinheit von wenigstens 99,8 Gew.-% p,p-BPA, niedriger Farbzahl und hoher Temperaturstabilität herstellen.

Nach der Phenolabtrennung auf Phenolgehalte von bevorzugt maximal 600 ppm, besonders bevorzugt maximal 100 ppm gemäß Schritt (g) erhält man eine Bisphenol A-Schmelze, welche ggf. ohne vorherige Verfestigung für die Herstellung von Polycarbonat nach dem Umesterungsverfahren (Schmelzepolycarbonat) verwendet werden kann. Die Bisphenol A-Schmelze kann aber auch durch bekannte Verfahren, wie dem Prillverfahren oder durch Abschuppung, für den Verkauf oder die Weiterverwertung verfestigt werden. Ferner kann die Schmelze in Natronlauge gelöst werden und für die Herstellung von Polycarbonat nach dem Phasengrenzflächenverfahren eingesetzt werden.

Dabei wird ein Polycarbonat mit einem niedrigen Yellowness Index (YI) von maximal 1,5 als Maß für die Farbe erhalten.

In einer anderen Ausführungsform des Verfahrens wird in Schritt (g) die Schmelze, wie in DE 199 54 787 A beschrieben, auf Restgehalte an Phenol von 2 bis 20 Gew.-% eingedampft und diese Schmelze ohne vorherige Verfestigung mit Diphenylcarbonat nach dem Schmelzeverfahren zu Polycarbonat umgesetzt werden. Auch hier wird ein Polycarbonat mit einem niedrigen YI erhalten.

Die bei der Fest-Flüssigtrennung in Schritt (c) anfallende Flüssigphase (Mutterlauge) enthält Phenol, BPA, Wasser sowie ggf. Aceton und Cokatalysator und ist angereichert an den bei der BPA-Herstellung typischerweise anfallenden Nebenkomponenten. Aus der in Schritt (c) erhaltene Flüssigphase (Mutterlauge) wird das Wasser auf Restgehalte von maximal 0,3 Gew.-%, bevorzugt 0,25 Gew.-%, abgetrennt und die erhaltene Lösung zu mindestens 90 Gew.-% in die Reaktion gemäß Schritt (a) zurückgeführt, wobei ggf. noch Aceton und Phenol und Cokatalysator zugesetzt werden.

Mit zunehmendem Konzentrationsniveau von Wasser und/oder ggf. Aceton in der zweiten Kristallisationsstufe (e) kann eine thermische Abtrennung dieser Komponenten aus der von Schritt (f) nach Schritt (c) überführten phenolischen Lösung sowie ggf. Rückführung des erhaltenen Destillates in den zweiten Kristallisationsschritt (in oder vor Schritt (e)) vorteilhaft sein, insbesondere, wenn die Konzentration dieser Verbindungen in der zweiten Kristallisationsstufe so hoch wird, dass eine separate Eindampfung bzw. Destillation der von Schritt (f) nach Schritt (c) überführten Lösungen als unscharfe Trennung im Vergleich zur Wasserabtrennung im Hauptkreislauf wirtschaftliche Vorteile bietet.

Nach der Aufarbeitung des Stoffstromes zwischen Schritt (c) und Schritt (a) zur Entfernung von Wasser wird diesem ein Teilstrom von 0,5 bis 10 Gew.-% entnommen, die sogenannte Ausspeisung. Dieser Teilstrom wird, gegebenenfalls nach Rückgewinnung von Phenol, Isopropenylphenol, Bisphenol A oder anderen Komponenten, ggf. nach vorheriger Behandlung mit Säure(n) und/oder Base(n), aus der Prozesskette als sogenanntes BPA-Harz ausgeschleust.

Die Ausspeisung kann beispielsweise zunächst einer Umlagerung an einem sauren Ionenaustauscher, dann einer teilweisen Phenoldestillation zur Aufkonzentrierung, anschließend einer Kristallisation und Filtration unterworfen werden. Das so gewonnene Destillat kann zur Filterkuchenwäsche und Tuchspülung genutzt werden. Das BPA-haltige Kristallisat kann zurück in die erste Kristallisationstufe (b) gefahren und die an Isomeren angereicherte Mutterlauge zur Rückgewinnung von weiterem Phenol weiter bevorzugt destillativ aufgearbeitet werden.

### Beispiele

### Beispiel 1 a): Ausführungsbeispiel

Ein gemäß Schritt (a) erhaltenes Produktgemisch, enthaltend 70,1 Gew.-% Phenol, 22,0 Gew.-% BPA, 6,6 Gew.-% Isomere, 1,1 Gew.-% Wasser und 0,2 Gew.-% Aceton wurde kontinuierlich einem Kristaller zum Zwecke der Suspensionskristallisation gemäß Schritt (b) zugeführt (1. Kristallisationsstufe). Die mittlere Verweilzeit in dem Kristaller betrug 1 h und wurde über einen externen Wärmetauscher umgewälzt. Die Temperatur des Kristallers wurde so auf 41°C eingestellt. Das unter stationären Bedingungen gefundene Kristallisat wies phenolfrei eine Zusammensetzung entsprechend Tabelle 1, Zeile 1 auf. Es wurde ein gedrungen nadelförmiger Kristallhabitus gefunden.

Die gemäß Schritt (c) abfiltrierten und gewaschenen BPA-Phenol-Adduktkristalle wurden geschmolzen und mit einer phenolischen Lösung gemäß Schritt (d) gemischt, so dass eine homogene Lösung, enthaltend 77 Gew.-% Phenol, 22 Gew.-% BPA und 1 Gew.-% Isomere entsprechend Tabelle 1, Zeile 2, entstand.

Diese homogene Lösung entsprechend Tabelle 1, Zeile 2 wurde mit Wasser im Verhältnis 99:1 gemischt und entsprechend den beschriebenen Bedingungen (Verweilzeit 1 h, externer Wärmetauscher, Temperatur 41°C) kristallisiert (2. Kristallisationsstufe, Schritt (e)). Die unter stationären Bedingungen nach Filtration und Wäsche gemäß Schritt (f) gefundenen BPA-Phenol-Adduktkristalle wiesen phenolfrei eine Zusammensetzung entsprechend Tabelle 1 Zeile 4 auf. Es wurde ein gedrungener, leicht nadelförmiger Kristallhabitus gefunden.

### Beispiel 1 b): Vergleichsbeispiel

Ein analog Schritt (a) erhaltenes Produktgemisch enthaltend, 70,1 Gew.-% Phenol, 22,0 Gew.-% BPA, 6,6 Gew.-% Isomere, 1,1 Gew.-% Wasser und 0,2 Gew.-% Aceton wurde kontinuierlich einem Kristaller zum Zwecke einer Suspensionskristallisation zugeführt (1. Kristallisationsstufe). Die mittlere Verweilzeit in dem Kristaller betrug 1 h und wurde über einen externen Wärmetauscher umgewälzt. Die Temperatur des Kristallers wurde so auf 41°C eingestellt. Das unter stationären Bedingungen gefundene Kristallisat wies phenolfrei eine Zusammensetzung entsprechend Tabelle 1, Zeile 1 auf. Es wurde ein gedrungen nadelförmiger Kristallhabitus gefunden.

Die abfiltrierten und gewaschenen BPA-Phenol-Adduktkristalle wurden geschmolzen und mit einer phenolischen Lösung gemischt, so dass eine homogene Lösung, enthaltend 77 Gew.-% Phenol, 22 Gew.-% BPA und 1 Gew.-% Isomere entsprechend Tabelle 1, Zeile 2, entstand.

Diese homogene Lösung wurde entsprechend den oben beschriebenen Bedingungen (Verweilzeit 1 h, externer Wärmetauscher, Temperatur 41°C) in einer Suspensionskristallisation ohne Anwesenheit von Wasser kristallisiert (2. Kristallisationsstufe). Die unter stationären Bedingungen nach Filtration und Wäsche gefundenen BPA-Phenol-Adduktkristalle wiesen phenolfrei eine Zusammensetzung entsprechend Tabelle 1, Zeile 3 auf. Es wurde ein ausgeprägt nadelförmiger Kristallhabitus gefunden. Die Produktreinheit war geringer als im erfindungsgemäßen Beispiel 1 a).

**Tabelle 1**

| Reinheit der BPA-Phenol-Adduktkristalle ohne Berücksichtigung des Phenolanteils sowie Zusammensetzung des Rekristallisationsfeed | | | |
|---|---|---|---|
| | Phenol [Gew.-%] | p,p-BPA [Gew.-%] | Isomere [Gew.-%] |
| 1. einfach kristallisiert | | 99,29 | 0,71 |
| 2. Rekristallisationsfeed | 77 | 22 | 1 |
| 3. rekristallisiert (Vergleichsbeispiel 1b)) | | 99,84 | 0,16 |
| 4. rekristallisiert, Wasserzusatz (Ausführungsbeispiel 1a)) | | 99,88 | 0,12 |

### Beispiel 2:

Eine phenolische Lösung, enthaltend 4 Gew-% Aceton, maximal 0,1 Gew.-% Wasser, 9 Gew.-% p,p'-BPA sowie Isomere, wurde in Schritt (a) mit 50 t/h in Gegenwart eines sulfonsauren Ionenaustauscherharzes und von Mercaptopropionsäure als Cokatalysator reagiert. Das Reaktionsprodukt enthielt etwa 24 Gew.-% p,p'-BPA.

Nach Zugabe eines an p,p'-BPA konzentrierten Teilstroms aus den Schritten (c) und (f) wurde aus dem resultierenden Gemisch BPA-Addukt in einer ersten Kristallisationsstufe (b) in zwei Schritten bei 54°C bzw. 41°C bei einer gesamten Verweilzeit von 8 h kontinuierlich kristallisiert, wobei die Kühlung der Suspension und Abfuhr der Kristallisationswärme mittels Wärmeaustauscher erfolgte.

Die Fest-Flüssig-Trennung der Suspension gemäß Schritt (c) erfolgte an einem Vakuumdrehfilter, wobei der abfiltrierte Filterkuchen mit phenolischer Lösung bei etwa 55°C gewaschen wurde.

Der Filterkuchen wurde zunächst aufgeschmolzen und anschließend mit einer phenolischen Lösung zu einer homogenen Lösung gemäß Schritt (d) vermischt. Die so hergestellte Lösung hatte einen Gehalt von 25 Gew.-% p,p'-BPA und 0,3 Gew.-% Isomeren. Vor der Kristallisation wurde Wasser zugegeben, so dass der Gehalt an Wasser in der homogenen Lösung 1 Gew.-% betrug. Die Temperatur der Lösung wurde auf 79°C eingestellt.

Die erneute Kristallisation.wurde gemäß Schritt (e) kontinuierlich in zwei Schritten bei 54°C und 41°C durchgeführt, wobei die Kühlung der Suspension und Abfuhr der Kristallisationswärme jeweils mittels Wärmeaustauscher erfolgte. Die Verweilzeit in dieser Kristallisationsstufe (e) betrug 4 h.

In Schritt (f) wurde die Suspension an einem Vakuumdrehfilter filtriert und mit phenolischer Lösung, enthaltend maximal 0,1 Gew.% Isomere, bei etwa 55°C gewaschen.

Die so gewonnenen phenol-feuchten BPA-Phenol-Addukt-Kristalle mit einer Restfeuchte von etwa 25 Gew.-% wurden anschließend geschmolzen und das Phenol gemäß Schritt (g) auf einen Gehalt von 90 ppm thermisch abgetrennt. Nach Prillen der BPA-Schmelze hatte das gewonnene BPA eine Reinheit von mindestens 99,9%, sowie eine Farbzahl von weniger als 20 Hazen.

Die Mutterlauge aus Schritt (c) und (f) wurde weitgehend entwässert. Aus der Mutterlauge wurde einTeilstrom entnommen und einer Phenoldestillation unterzogen. Das Destillat wurde zur Filterkuchenwäsche und Tuchspülung genutzt. Aus dem an BPA angereicherten Sumpfprodukt wurde nach weiterer Auftrennung mittels Kristallisation und Filtration ein BPA-haltiger Strom zurück in die erste Kristallisationstufe (b) gefahren und die an Isomeren angereicherte Mutterlauge zur Rückgewinnung von Phenol weiter aufgearbeitet.

### Beispiel 3: Simulation eines zweistufigen Kristallisationsverfahrens

Im Folgenden wird eine Simulationsstudie für ein zweistufiges Kristallisationsverfahren zur Herstellung von hochreinem Bisphenol A beschrieben. Die Simulationen wurden mit der kommerziellen Softwareumgebung Aspen Custom Modeler^{®} durchgeführt. Der Software liegt ein Verfahrensmodell mit speziellen Modellen für die Reaktion (detaillierte Reaktionskinetik), für die Kinetik des Einbaus von Verunreinigungen bei der Kristallisation sowie für weitere Grundoperationen zugrunde.
- Schritt (a):: Reaktion von Aceton und Phenol zu p,p'-BPA, Isomeren unter Bildung von Wasser in adiabaten Festbettreaktoren in Anwesenheit eines Cokatalysators bei folgenden Zulaufbedingungen:
4 Gew.-% Aceton, 0,07 Gew.-% Wasser, 9 Gew.-% p,p'-BPA; Temperatur: 52°C
- Schritt (b):: Die Reaktionsprodukte, enthaltend 22,5 Gew.-% p,p'-BPA, wurden in die erste Kristallisationsstufe geleitet, wo BPA-Addukt bei einer gesamten Verweilzeit von 5 h in zwei Schritten bei 54°C bzw. 41°C kristallisiert wurde. Die berechnete Reinheit der Kristalle betrug 99,68 Gew.-% bezogen auf Bisphenol A.
- Schritt (c):: Die Suspension wurde über ein Vakuumdrehfilter filtriert, gewaschen und entfeuchtet. Zur Wäsche des Filterkuchens wurde ein Teilstrom von 27 % der Filtrate aus Schritt (f) eingesetzt, was einem Verhältnis von Menge der Waschflüssigkeit zu Menge des gewonnenen Feststoffs von 0,9 entsprach.

Die aus der Umkristallisationsstufe ausgeschleuste phenolische Mutterlauge enthielt 9 Gew.-% p,p'-BPA, 0,3 Gew.-% Isomere und 1,3 Gew.-% Wasser. Ein Teil dieses Teilstroms wurde zusammen mit Rückphenolen aus der Anlage zur Reinigung des Filtertuchs genutzt.

Der entfeuchtete Filterkuchen besaß eine Restfeuchte von 25 Gew.-%. Mit den Verunreinigungen aus der Mutterlauge, die noch an den Kristallen anhaftete, sowie aus der an Isomeren beladenen Waschflüssigkeit lag die Reinheit des Filterkuchens nach Austritt aus dem Drehfilter bei 99,4 Gew.-% bezogen auf p,p'-BPA.
- Schritt (d):: Der Filterkuchen aus (c) wurde aufgeschmolzen und mit den restlichen Filtraten aus (f) vermischt, derart, dass die Konzentration an p,p'-BPA im Zulauf der zweiten Kristallisationsstufe (e) 23 Gew.-% beträgt. Die entstandene Lösung enthielt weiterhin 0,3 Gew.-% Isomere sowie 1 Gew.-% Wasser.
- Schritt (e):: Kristallisation in zwei Schritten bei 54°C/41°C bei einer gesamten Verweilzeit von 5,5 h. Die berechnete Reinheit der Adduktkristalle betrug 99,93 Gew.-% bezogen auf p,p'-BPA.
- Schritt (f):: Die erhaltene Suspension wurde filtriert, mit Frischphenol sowie rückgewonnenen Phenolströmen aus der Anlage gewaschen und anschließend entfeuchtet. Ein Teil dieser Phenolströme wurde ebenfalls zur Reinigung des Filtertuchs genutzt. Die zur Filterkuchenwäsche eingesetzte Waschflüssigkeitsmenge betrug 71 Gew.-% des gewonnenen Feststoffs. Die in Schritt (f) angefallenen Filtrate aus Filtration und Wäsche wurden vereinigt und in die Schritte (c) und (d) rückgeführt.
- Schritt (g):: Der Filterkuchen mit einer Restfeuchte von 25 Gew.-% wurde aufgeschmolzen. Der Gehalt an Phenol wurde mittels thermischer Trennung auf einen Restgehalt von 150 ppm in der BPA-Schmelze verringert. Das gewonnene Phenol wurde in Schritt (f) geleitet. Die gewonnene BPA-Schmelze besaß eine Reinheit von 99,92 Gew.-% bezogen auf p,p'-BPA.

Die Mutterlauge aus Schritt (c) wurde auf einen Wassergehalt von 0,075 Gew.-% entwässert. Daraus wurde ein Teilstrom entsprechend 4 % der Gesamtmenge zur Ausschleusung der Isomere als BPA-Harz nach einer nachgeschalteten Phenolrückgewinnung abgezogen. Der übrige Teil der entwässerten Mutterlauge wurde unter Zusatz von Aceton, Phenol sowie eines Cokatalysators zurück in die Reaktion gemäß Schritt (a) geleitet.

Gemäß Simulationsstudie verringerte sich der Verunreinigungsgehalt im Produkt um 12 %, wenn in Schritt (f) die Filtrate aus Filtration und Wäsche nicht vereinigt wurden, sondern derart getrennt wurden, dass die stärker mit Isomeren beladene Mutterlauge (Filtrat aus der Filtration) bevorzugt in Schritt (c) und die Waschfiltrate aus (f) in Schritt (d) geleitet wurden.

## Patentansprüche

1. Verfahren zur Herstellung von Bisphenol A umfassend die folgenden Schritte:
a) Umsetzung von Phenol und Aceton in Gegenwart eines sulfonsauren Ionenaustauschers und eines schwefelhaltigen Cokatalysators zu einem Bisphenol A enthaltenden Produktgemisch
b) kontinuierliche Suspensionskristallisation eines Bisphenol A-Phenol-Addukts aus dem in Schritt (a) erhaltenen Produktgemisch, welches 0,1 bis 6 Gew.%, bevorzugt 0,5 bis 2 Gew.%, Wasser enthält, in mindestens einem Kristallisator mit jeweils mindestens einem Wärmetauscher
c) Abtrennung der gemäß Schritt (b) gewonnenen Bisphenol A-Phenol-Adduktkristalle durch Fest-Flüssig-Trennung, insbesondere durch Filtration, und Waschen der Feststoffphase, insbesondere des Filterkuchens, mit phenolischer Lösung sowie anschließende destillative Abtrennung von Wasser aus den Flüssigphasen, insbesondere aus den Filtraten, der Fest-Flüssig-Trennung und/oder der Wäsche auf einen Gehalt von maximal 0,3 Gew.-% Wasser und Rückführung der so entwässerten Lösung zu mindestens 90 Gew.-% in Schritt (a) (Teilstrom A), wobei die rückgeführte Lösung einen Gehalt von 5 bis 15 Gew.%, bevorzugt 6,5 bis 10 Gew.%, p,p'-BPA und 3 bis 12 Gew.% Isomere aufweist, und der restliche Teilstrom B von 0,5 bis 10 Gew.-% ausgeschleust wird.
d) Herstellen einer homogenen Lösung, enthaltend 15 bis 35 Gew.%, bevorzugt 20 bis 30 Gew.%, Bisphenol A, 0,05 bis 2 Gew.%, bevorzugt 0,1 bis 1,1 Gew.%, Isomere und 0,1 bis 10 Gew.% Wasser, in Phenol aus der in Schritt (c) erhaltenen Feststoffphase, insbesondere aus dem Filterkuchen, unter Zugabe einer phenolischen Lösung und Wasser oder einer Wasser enthaltenden phenolischen Lösung.
e) kontinuierliche Suspensionskristallisation eines Bisphenol A-Phenol-Addukts aus der in Schritt (d) erhaltenen Lösung in mindestens einem Kristallisator mit jeweils mindestens einem Wärmetauscher
f) Abtrennung der gemäß Schritt (e) gewonnenen Bisphenol A-Phenol-Adduktkristalle durch Fest-Flüssig-Trennung, insbesondere durch Filtration, und Waschen der Feststoffphase, insbesondere des Filterkuchens, mit phenolischer Lösung
g) Entfernung von Phenol aus dem gemäß Schritt (f) erhaltenen BPA-Phenol-Addukt durch Erhitzen auf Temperaturen von mindestens 120°C.

2. Verfahren nach Anspruch 1, **dadurch gekennzeichnet, dass** der Teilstrom B gegebenenfalls nach Rückgewinnung von Phenol, Isopropenylphenol, Bisphenol A oder anderen Komponenten, gegebenenfalls nach einer vorherigen Behandlung mit Säuren und / oder Basen, aus der Prozesskette ausgeschleust wird.

3. Verfahren nach Anspruch 2, **dadurch gekennzeichnet, dass** ggf. nach vorheriger Behandlung durch Säuren aus dem Teilstrom B Phenol durch Destillation und / oder Bisphenol A/Phenol-Addukt durch Kristallisation zurückgewonnen werden und der verbleibende Teilstrom B ansschließend ggf, mit einer Säuren versetzt und erneut destillativ Phenol zurückgewonnen wird.

4. Verfahren nach Anspruch 2 oder 3, **dadurch gekennzeichnet, dass** das aus dem Teilstrom B rückgewonnene, aufgereinigte Phenol zur Filterkuchenwäsche und Tuchspülung genutzt wird.

5. Verfahren nach einem der Ansprüche 1-4, **dadurch gekennzeichnet, dass** 50 bis 90 Gew.-%, bevorzugt 60 bis 80 Gew.%, der in Schritt (f) erhaltenen Flüssigphase in Schritt (d) eingesetzt werden.

6. Verfahren nach einem der Ansprüche 1-5, **dadurch gekennzeichnet, dass** 10 bis 50 Gew.-%, bevorzugt 20 bis 40 Gew.-% der in Schritt (f) erhaltenen Flüssigphase zur Durchführung der Wäsche in Schritt (c) rückgeführt werden.

7. Verfahren nach einem der Ansprüche 1-6, **dadurch gekennzeichnet, dass** das in Schritt (f) eingesetzte Waschphenol maximal 1,0 Gew.% an weiteren phenolischen Komponenten enthält.

8. Verfahren nach einem der Ansprüche 1-7, **dadurch gekennzeichnet, dass** zur Herstellung der homogenen Lösung in Schritt (d) zunächst die in Schritt (c) erhaltenen BPA-Phenol-Adduktkristalle aufgeschmolzen und anschließend mit einer phenolischen Lösung vermischt werden.

9. Verfahren nach einem der Ansprüche 1-8, **dadurch gekennzeichnet, dass** in Schritt (c) und/oder in Schritt (f) ein Vakuumdrehfilter zur Fest-Flüssigtrennung verwendet wird.

10. Verfahren nach einem der Ansprüche 1-9, **dadurch gekennzeichnet, dass** die in Schritt (c) verwendete Waschflüssigkeit 84 bis 99,45 Gew.% Phenol, 0,5 bis 15 Gew.% BPA, 0,05 bis 1,0 Gew.-% Isomere, bezogen auf die Lösung ohne Berücksichtigung eines Anteils an Wasser, enthält.

11. Verfahren nach einem der Ansprüche 1-10, **dadurch gekennzeichnet, dass** die in Schritt (c) verwendete Waschflüssigkeit 0,05 bis 12 Gew.% Wasser, bevorzugt 0,2 bis 3 Gew.-%, Wasser bezogen auf die Gesamtmenge, enthält.

12. Verfahren nach einem der Ansprüche 1-11, **dadurch gekennzeichnet, dass** die in Schritt (c) verwendete Waschflüssigkeit zu 5 bis 100 Gew.% aus Schritt (f) stammt.

13. Verfahren nach einem der Ansprüche 1-12, **dadurch gekennzeichnet, dass** in der ersten Kristallisationsstufe (c) seriell in zwei Schritten bei verschiedenen Temperaturen, in der zweiten Kristallisationsstufe (e) in einem Schritt in einem oder mehreren parallel betriebenen Kristallern kristallisiert wird.

14. Verfahren nach einem der Ansprüche 1-13, **dadurch gekennzeichnet, dass** die in Schritt (e) eingetragene Lösung aus Schritt (d) auf einen Acetongehalt von 0 bis 5 Gew.% Aceton eingestellt wird.

15. Verfahren nach einem der Ansprüche 1-14, **dadurch gekennzeichnet, dass** die Flüssigphase der Fest-Flüssig-Trennung und der Wäsche gemäß Schritt (f) wenigstens teilweise getrennt voneinander aufgefangen, die Flüssigphase der Fest-Flüssig-Trennung wenigstens teilweise in Schritt (c) zurückgeführt und die Flüssigphase der Wäsche bevorzugt wenigstens teilweise in Schritt (d) zurückgeführt werden.

16. Verfahren nach einem der Ansprüche 1-15, **dadurch gekennzeichnet, dass** 0 bis 35 Gew.-%, bevorzugt 0 bis 10 Gew.%, der in Schritt (f) anfallenden Flüssigphasen eingedampft werden, das Kopfprodukt in Schritt (d) und/oder Schritt (f) geleitet und das Sumpfprodukt vor Schritt (b) zurückgeführt wird.

## Claims

1. Process for the preparation of bisphenol A comprising the following steps:
a) reaction of phenol and acetone in the presence of an ion exchanger in sulfonic acid form and a sulfur-containing co-catalyst to give a product mixture containing bisphenol A;
b) continuous suspension crystallization of a bisphenol A-phenol adduct from the product mixture obtained in step (a), which contains 0.1 to 6 wt.% and preferably 0.5 to 2 wt.% of water, in at least one crystallizer each having at least one heat exchanger;
c) solid-liquid separation of the crystals of bisphenol A-phenol adduct obtained in step (b), especially by filtration, and washing of the solid phase, especially the filter cake, with a phenolic solution, followed by distillative separation of water from the liquid phases, especially the filtrates, from the solid-liquid separation and/or the washing to a maximum water content of 0.3 wt.%, and recycling of at least 90 wt.% of the dewatered solution into step (a) (partial stream A), the recycled solution containing 5 to 15 wt.% and preferably 6.5 to 10 wt.% of p,p'-BPA and 3 to 12 wt.% of isomers and the remaining partial stream B of 0.5 to 10 wt.% being discharged;
d) preparation of a homogeneous phenolic solution, containing 15 to 35 wt.% and preferably 20 to 30 wt.% of bisphenol A, 0.05 to 2 wt.% and preferably 0.1 to 1.1 wt.% of isomers and 0.1 to 10 wt.% of water, from the solid phase obtained in step (c), especially the filter cake, with the addition of a phenolic solution and water or a water-containing phenolic solution;
e) continuous suspension crystallization of a bisphenol A-phenol adduct from the solution obtained in step (d) in at least one crystallizer each having at least one heat exchanger;
f) solid-liquid separation of the crystals of bisphenol A-phenol adduct obtained in step (e), especially by filtration, and washing of the solid phase, especially the filter cake, with a phenolic solution; and
g) removal of phenol from the BPA-phenol adduct obtained in step (f) by heating to temperatures of at least 120°C.

2. Process according to Claim 1, **characterized in that** the partial stream B is discharged from the process chain, optionally after recovery of phenol, isopropyl phenol, bisphenol A or other components, optionally after previous treatment with acids and/or bases.

3. Process according to Claim 2, **characterized in that** phenol is recovered from the partial stream B by distillation and/or bisphenol A/phenol adduct is recovered by crystallization, optionally after previous treatment with acids, and an acids is then optionally added to the remaining partial stream B and phenol is again recovered by distillation.

4. Process according to Claim 2 or 3, **characterized in that** the purified phenol recovered from the partial stream B is used for washing the filter cake and rinsing the cloth.

5. Process according to one of Claims 1-4, **characterized in that** 50 to 90 wt.%, preferably 60 to 80 wt.%, of the liquid phase obtained in step (f) is used in step (d).

6. Process according to one of Claims 1-5, **characterized in that** 10 to 50 wt.%, preferably 20 to 40 wt.%, of the liquid phase obtained in step (f) is recycled into step (c) for washing purposes.

7. Process according to one of Claims 1-6, **characterized in that** the washing phenol used in step (f) contains a maximum of 1.0 wt.% of other phenolic components.

8. Process according to one of Claims 1-7, **characterized in that**, to prepare the homogeneous solution in step (d), the crystals of BPA-phenol adduct obtained in step (c) are first melted and then mixed with a phenolic solution.

9. Process according to one of Claims 1-8, **characterized in that** a vacuum rotary filter is used for the solid-liquid separation in step (c) and/or step (f).

10. Process according to one of Claims 1-9, **characterized in that** the washing liquid used in step (c) contains 84 to 99.45 wt.% of phenol, 0.5 to 15 wt.% of BPA and 0.05 to 1.0 wt.% of isomers, based on the solution without taking water into account.

11. Process according to one of Claims 1-10, **characterized in that** the washing liquid used in step (c) contains 0.05 to 12 wt.% of water, preferably 0.2 to 3 wt.% of water, based on the total amount.

12. Process according to one of Claims 1-11, **characterized in that** 5 to 100 wt.% of the washing liquid used in step (c) originates from step (f).

13. Process according to one of Claims 1-12, **characterized in that** crystallization takes place in the first crystallization stage, (c), in two steps in series at different temperatures, and in the second crystallization stage, (e), in one step in one or more crystallizers operated in parallel.

14. Process according to one of Claims 1-13, **characterized in that** the solution from step (d) introduced into step (e) is adjusted to an acetone content of 0 to 5 wt.%.

15. Process according to one of Claims 1-14, **characterized in that** at least part of the liquid phase from the solid-liquid separation and at least part of the liquid phase from the washing in step (f) are collected separately, at least part of the liquid phase from the solid-liquid separation is recycled into step (c) and preferably at least part of the liquid phase from the washing is recycled into step (d).

16. Process according to one of Claims 1-15, **characterized in that** 0 to 35 wt.%, preferably 0 to 10 wt.%, of the liquid phases obtained in step (f) is evaporated, the top product is passed to step (d) and/or step (f) and the bottom product is recycled upstream of step (b).

## Revendications

1. Procédé de préparation de bisphénol A comprenant les étapes suivantes:
a) transformation de phénol et d'acétone en présence d'un échangeur d'ions à base d'acide sulfurique et d'un co-catalyseur sulfuré en un mélange de produits contenant du bisphénol A;
b) cristallisation en suspension continue d'un adduit de bisphénol A-phénol provenant du mélange de produits obtenus lors de l'étape a), qui contient 0,1 à 6 % en poids, de préférence 0,5 à 2 % en poids d'eau, dans au moins un cristalliseur comportant au moins un échangeur de chaleur;
c) séparation des cristaux d'adduit de bisphénol A-phénol obtenus conformément à l'étape b) par séparation solide-liquide, en particulier par filtration, et lavage de la phase solide, en particulier du gâteau de filtration, avec une solution phénolique, suivie d'une séparation par distillation de l'eau provenant des phases liquides, en particulier des produits de filtration, de la séparation solide-liquide et/ou du lavage jusqu'à une teneur maximale de 0,3 % en poids d'eau et recyclage de la solution ainsi déshydratée à au moins 90 % en poids dans l'étape a) (courant partiel A), la solution recyclée présentant une teneur comprise entre 5 et 15 % en poids, de préférence entre 6,5 et 10 % en poids de p,p'-BPA et de 3 à 12 % en poids d'isomères, et le courant partiel restant B de 0,5 à 10 % en poids étant éclusé;
d) préparation d'une solution homogène contenant 15 à 35 % en poids, de préférence 20 à 30 % en poids de bisphénol A, 0,05 à 2 % en poids, de préférence 0,1 à 1,1 % en poids d'isomères et 0,1 à 10 % en poids d'eau, dans du phénol provenant de la phase solide obtenue lors de l'étape c), en particulier du gâteau de filtration, en ajoutant une solution phénolique et de l'eau ou une solution phénolique contenant de l'eau.
e) cristallisation en suspension continue d'un adduit de bisphénol A-phénol provenant de la solution obtenue lors de l'étape d) dans au moins un cristalliseur comportant au moins un échangeur de chaleur;
f) séparation des cristaux d'adduit de bisphénol A-phénol obtenus conformément à l'étape e) par séparation solide-liquide, en particulier par filtration, et lavage de la phase solide, en particulier du gâteau de filtration, avec une solution phénolique;
g) élimination du phénol de l'adduit de BPA-phénol obtenu conformément à l'étape f) par chauffage à des températures d'au moins 120°C.

2. Procédé selon la revendication 1, **caractérisé en ce que** le courant partiel B est éclusé de la chaîne de processus, le cas échéant après récupération du phénol, de l'isopropénylphénol, du bisphénol A ou d'autres composants, le cas échéant après un traitement préalable par des acides et/ou des bases.

3. Procédé selon la revendication 2, **caractérisé en ce que**, le cas échéant après un traitement préalable par des acides provenant du courant partiel B, le phénol est récupéré par distillation et/ou l'adduit de bisphénol A-phénol est récupéré par cristallisation, et le courant partiel B restant est ensuite mélangé le cas échéant avec un acide et du phénol est à nouveau récupéré par distillation.

4. Procédé selon la revendication 2 ou 3, **caractérisé en ce que** le phénol récupéré du courant partiel B et purifié est utilisé pour le lavage du gâteau de filtration et le rinçage du tissu filtrant.

5. Procédé selon une des revendications 1 à 4, **caractérisé en ce que** 50 à 90 % en poids, de préférence 60 à 80 % en poids de la phase liquide obtenue lors de l'étape f) sont utilisés dans l'étape d).

6. Procédé selon une des revendications 1 à 5, **caractérisé en ce que** 10 à 50 % en poids, de préférence 20 à 40 % en poids de la phase liquide obtenue lors de l'étape f) sont recyclés pour effectuer le lavage dans l'étape c).

7. Procédé selon une des revendications 1 à 6, **caractérisé en ce que** le phénol de lavage utilisé dans l'étape f) contient au maximum 1,0 % en poids d'autres composants phénoliques.

8. Procédé selon une des revendications 1 à 7, **caractérisé en ce que** pour la préparation de la solution homogène lors de l'étape d), les cristaux d'adduits de BPA-phénol obtenus lors de l'étape c) sont tout d'abord fondus puis mélangés avec une solution phénolique.

9. Procédé selon une des revendications 1 à 8, **caractérisé en ce qu'**un filtre rotatif sous vide est utilisé pour la séparation solide-liquide lors de l'étape c) et/ou de l'étape f).

10. Procédé selon une des revendications 1 à 9, **caractérisé en ce que** le liquide de lavage utilisé lors de l'étape c) contient 84 à 99,45 % en poids de phénol, 0,5 à 15 % en poids de BPA et 0,05 à 1,0 % en poids d'isomères par rapport à la solution sans tenir compte d'une fraction d'eau.

11. Procédé selon une des revendications 1 à 10, **caractérisé en ce que** le liquide de lavage utilisé lors de l'étape c) contient 0,05 à 12 % en poids d'eau, de préférence 0,2 à 3 % en poids d'eau par rapport au volume total.

12. Procédé selon une des revendications 1 à 11, **caractérisé en ce que** le liquide de lavage utilisé lors de l'étape c) provient de l'étape f) à raison de 5 à 100 % en poids.

13. Procédé selon une des revendications 1 à 12, **caractérisé en ce que** dans la première étape de cristallisation c), la cristallisation est opérée en série en deux phases à différentes températures et dans la deuxième étape de cristallisation e), en une phase dans un ou plusieurs cristalliseurs opérés en parallèle.

14. Procédé selon une des revendications 1 à 13, **caractérisé en ce que** la solution provenant de l'étape d) introduite dans l'étape e) est ajustée à une teneur en acétone comprise entre 0 et 5 % en poids d'acétone.

15. Procédé selon une des revendications 1 à 14, **caractérisé en ce que** la phase liquide de la séparation solide-liquide et celle du lavage selon l'étape f) sont recueillies au moins en partie séparément l'une de l'autre, la phase liquide de la séparation solide-liquide est recyclée au moins en partie dans l'étape c) et la phase liquide du lavage est recyclée dans l'étape d) au moins en partie.

16. Procédé selon une des revendications 1 à 15, **caractérisé en ce que** 0 à 35 % en poids, de préférence 0 à 10 % en poids des phases liquides se présentant lors de l'étape f) sont concentrés par évaporation, le produit de tête est acheminé dans l'étape d) et/ou l'étape f) et le produit de bas de colonne est recyclé avant l'étape b).
